# EUROPÄISCHE PATENTSCHRIFT

(11) **EP 3 793 486 B1**
(45) Veröffentlichungstag und Bekanntmachung des Hinweises auf die Patenterteilung: **07.12.2022**
(21) Anmeldenummer: 19724794.3
(22) Anmeldetag: 14.05.2019
(51) Int. Cl.: A61F 2/64

(54) **VENTIL UND PROTHESENKNIEGELENK MIT EINEM SOLCHEN**
VALVE AND PROSTHETIC KNEE JOINT HAVING SUCH A VALVE
SOUPAPE ET PROTHÈSE DE L'ARTICULATION DU GENOU COMPRENANT UNE TELLE SOUPAPE

(30) Priorität: 14.05.2018 DE 102018111441
(43) Veröffentlichungstag der Anmeldung: 24.03.2021
(73) Patentinhaber: Ottobock SE & Co. KGaA, 37115 Duderstadt (DE)
(72) Erfinder: BOITEN, Herman, 6715 LE Ede (NL)
(74) Vertreter: Gramm, Lins & Partner Patent- und Rechtsanwälte PartGmbB
(86) Internationale Anmeldenummer: PCT/EP2019/062366
(87) Internationale Veröffentlichungsnummer: WO 2019/219697

(56) Entgegenhaltungen:
- EP-A2- 2 000 714
- DE-A1- 4 233 247
- DE-A1-102007 032 090

## Beschreibung

Die Erfindung betrifft ein Prothesenkniegelenk mit einem Ventil mit einem Zulauf, einem Ablauf, der mit dem Zulauf durch eine Fluidverbindung verbunden ist, und einen Ventilkörper, der durch Verschieben entlang einer Verschieberichtung in eine erste Position, in der die Fluidverbindung blockiert ist, und in eine zweite Position bringbar ist, in der die Fluidverbindung geöffnet ist, wobei der Zulauf des Ventils mit der Flexionskammer und der Ablauf des Ventils mit der Extensionskammer verbunden sind.

Prothesenkniegelenke und dafür verwendbare Hydraulikventile sind aus dem Stand der Technik seit langem bekannt. Prothesenkniegelenke sollten in unterschiedlichen Phasen eines Schrittzyklus unterschiedlich stark gedämpft sein. Während in der Schwungphase eine geringe Dämpfung nötig ist, ist in der Standphase eine höhere Dämpfung wünschenswert. Am Anfang der Standphase, bei Fersenauftritt, ist das Prothesenknie in der Regel vollständig gestreckt. In diesem Fall ist es wichtig, einer möglichen Flexion des Knies einen hohen Widerstand entgegenzusetzen, um ein stabiles und sicheres Trage- und Gehgefühl dem Träger der Prothese zu vermitteln. Dies gilt jedoch nicht nur im vollständig gestreckten Zustand. Stolpert der Träger der Prothese beispielsweise, wird der vollständig gestreckte Zustand des Knies in der Regel nicht erreicht, bevor der Fuß auf den Boden aufsetzt. Dennoch muss gerade in diesen Fällen eine unkontrollierte und zu leichtgängige weitere Flexion des Knies vermieden werden, um einen Sturz des Trägers zu vermeiden.

Aus der EP 2 000 714 A2 ist ein Ventil bekannt, das verwendet werden kann, wenn eine Öffnung des Ventils nur für einen sehr kurzen Zeitraum gewünscht ist. Das bewegliche Teil in dem dort gezeigten Ventil bewegt sich aus der geschlossenen Stellung in eine offene Stellung und weiter in eine zweite geschlossene Stellung, so dass keine Umkehr der Bewegungsrichtung notwendig ist

Aus der US 9,402,748 B2 ist daher beispielsweise ein Ventil bekannt, das den Strömungswiderstand in der Fluidverbindung nur dann reduziert, wenn neben einer Vorfußlast, also einer Belastung des Zehenbereichs des Fußes auch ein vollständig gestrecktes Knie detektiert wird. Während die Vorfußlast anzeigt, dass im Schrittzyklus als nächstes die Loslösung des Fußes vom Boden, das sogenannte "toe-off" stattfindet, ist dies als einzelner Steuerparameter nicht genug. Eine entsprechende Belastung des Vorfußes kann beispielsweise auch stattfinden, wenn der Träger der Prothese eine Rampe oder eine Neigung hinab geht und in gebeugtem Zustand der Vorfuß belastet wird. In diesem Fall darf jedoch anders als bei der beginnenden Schwungphase keinesfalls die Dämpfung des Kniegelenkes reduziert werden, da dies einen Sturz des Trägers zur Folge hätte. Daher wird als zweiter Steuerparameter die Streckung des Knies detektiert.

Derartige Knie mit doppelter Sensorik sind aufwendig, fehleranfällig und kostenintensiv herzustellen. Die unterschiedlichen Sensoren müssen zudem entweder mit elektrischen Strom versorgt werden, wodurch die Konstruktion des Knies groß, unhandlich und schwer wird, oder es müssen stromlos zu betreibende Sensoren verwendet werden, die konstruktiv aufwendig sind. Der Erfindung liegt daher die Aufgabe zugrunde, ein Ventil und ein Prothesenkniegelenk zu entwickeln, mit dem diese Nachteile vermieden oder zumindest reduziert werden. Die Erfindung löst die gestellte Aufgabe durch ein Ventil gemäß dem Oberbegriff des Anspruchs 1, das sich dadurch auszeichnet, dass der Zulauf derart ausgebildet und angeordnet ist, dass ein durch den Zulauf eintretendes Fluid eine Gesamtkraft auf den Ventilkörper ausübt, die zumindest auch in einer Kraftrichtung wirkt, die senkrecht auf der Verschieberichtung steht, wenn sich der Ventilkörper in der ersten Position befindet.

Wird ein derartiges Ventil in ein Prothesenkniegelenk eingebaut, kann es eine Fluidverbindung zwischen einer Extensionskammer und einer Flexionskammer des Hydrauliksystems des Prothesenkniegelenkes blockieren oder zumindest den Durchfluss reduzieren. Handelt es sich bei der Fluidverbindung, in die das Ventil eingesetzt wird, um die Fluidverbindung zwischen der Extensionskammer und der Flexionskammer des Prothesenkniegelenkes, hat dies bei einem geschlossenen Ventil, wenn sich also der Ventilkörper in der ersten Position befindet, eine vollständige Blockierung des Kniegelenks zur Folge. Vorzugsweise ist wenigstens eine weitere Fluidverbindungen vorhanden, die einen Durchfluss von Fluid aus der Extensionskammer in die Flexionskammer erlaubt. Dazu kann beispielsweise eine Bohrung in einem Kolben, beispielsweise einem Drehkolben, vorhanden sein, durch dessen Bewegung Fluid von einer Kammer in die andere Kammer befördert wird. Der Durchfluss ist vorzugsweise mit einem Ein-Weg-Ventil ausgestattet, um einen Rückfluss des Fluids aus der Flexionskammer in die Extensionskammer zu verhindern.

Versucht der Träger des Prothesenkniegelenkes in diesem Zustand des Ventils das Knie zu bewegen, also einen Oberschenkelteil des Knies relativ zu einem Unterschenkelteil des Knies zu verschwenken, entsteht in einem Teil des Hydrauliksystems des Prothesenkniegelenkes ein erhöhter Druck. Eine derartige Verschwenkung des Oberschenkelteils des Knies relativ zum Unterschenkelteil des Knies hat immer eine Verlagerung von Hydraulikflüssigkeit von der Extensionskammer in die Flexionskammer oder umgekehrt zur Folge. Da dies über eine Fluidverbindung geschieht, in der sich das Ventil befindet, wird dies zumindest teilweise durch das Ventil verhindert, solange sich der Ventilkörper in der ersten Position befindet. Das Fluid tritt folglich durch den Zulauf in das Ventil ein, kann es jedoch nicht verlassen, so dass das Fluid eine Gesamtkraft auf den Ventilkörper ausübt. Herkömmlicherweise wird bei Ventilen darauf geachtet, dass diese durch das Fluid ausgebübte Gesamtkraft keine Komponente aufweist, die nicht parallel zur Verschieberichtung des Ventilkörpers verläuft. Auf diese Weise wird eine hydrostatische Lagerung realisiert, die ein leichtes und verschleißarmes Verschieben des Ventils sicherstellt.

Eine entsprechende Ausgestaltung ist beispielsweise aus der US 7,344,569 B2 bekannt. Hier sind beispielsweise acht äquidistant über den Umfang des Ventils verteilte Öffnungen vorhanden, durch die Fluid, das durch den Zulauf in das Ventil eingetreten ist, auf den Ventilkörper trifft. Daher wird in diesem Fall durch das Fluid eine Gesamtkraft auf den Ventilkörper aufgetragen, die keine Komponente aufweist, die senkrecht auf der Verschieberichtung steht.

Dies ist bei einem erfindungsgemäßen Prothesenkniegelenk mit dem darin enthaltenen Ventil fundamental anders. Das durch den Zulauf eingetretene Fluid übt eine Gesamtkraft auf den Ventilkörper auf, die eine entsprechende Komponente aufweist, die senkrecht auf der Verschieberichtung steht.

Vorteilhafterweise liegt der Ventilkörper in dieser Kraftrichtung an einer Gehäusewand an. Durch die Gesamtkraft, die durch das Fluid auf den Ventilkörper aufgebracht wird, wenn sich dieser in der ersten Position befindet, kommt es zwischen dem Ventilkörper und der Gehäusewand, an der er anliegt, zu einer erhöhten Reibung. Es entsteht folglich eine Haltekraft, die einer Verschiebung des Ventilkörpers aus der ersten Position in die zweite Position entgegenwirkt. Diese Haltekraft tritt immer dann auf, wenn sich der Ventilkörper in der ersten Position befindet und gleichzeitig das Fluid eine Gesamtkraft auf den Ventilkörper ausübt. Diese Gesamtkraft kann nur dann auftreten, wenn ein Drehmoment auf das Knie wirkt, also eine Kraft vorhanden ist, die einen Unterschenkelteil des Prothesenkniegelenkes relativ zu einem Oberschenkelteil des Prothesenkniegelenkes verschwenken möchte. Dies ist jedoch nur im nicht vollständig gestreckten Zustand des Knies der Fall.

Durch die erfindungsgemäße Ausgestaltung eines Ventils lässt sich folglich der Sensor eines herkömmlichen Prothesenkniegelenkes, der die Streckung des Knies detektiert, vollständig vermeiden. Dadurch wird das Gesamtsystem kleiner, einfacher herzustellen und kostengünstiger.

Vorzugsweise verfügt das Ventil über eine bereits beschriebene Fluidverbindung zwischen einer Extensionskammer und einer Flexionskammer des Hydrauliksystems, die einen Durchfluss von Fluid aus der Extensionskammer in die Flexionskammer erlaubt. Dazu kann beispielsweise eine Bohrung in einem Kolben, beispielsweise einem Drehkolben, vorhanden sein, durch dessen Bewegung Fluid von einer Kammer in die andere Kammer befördert wird. Der Durchfluss ist vorzugsweise mit einem Ein-Weg-Ventil ausgestattet, um einen Rückfluss des Fluids aus der Flexionskammer in die Extensionskammer zu verhindern. Vorteilhafterweise ist der Zulauf derart ausgebildet, dass eintretendes Fluid den Ventilkörper entlang eines Abschnittes seines Umfangs anströmt, der kleiner als 240°bevorzugt kleiner als 210° und größer als 120°, bevorzugt größer als 150° ist. Besonders bevorzugt ist der Abschnitt 180°. Der Zulauf kann beispielsweise in Form eines Schlitzes vorhanden sein, der sich über den genannten Abschnitt des Umfanges beispielsweise eines den Ventilkörper umgebenden Gehäuses erstreckt. Durch die Ausgestaltung dieses Schlitzes in einem Winkelbereich wird die gewünschte Gesamtkraft auf der gegenüberliegenden Fläche erreicht.

In einer bevorzugten Ausgestaltung verfügt das Ventil über einen Schaltstift, durch dessen Betätigen der Ventilkörper von der ersten Position in die zweite Position bringbar ist. Ein solcher Schaltstift kann beispielsweise verwendet werden, um den Ventilkörper von der ersten Position in die zweite Position zu verschieben und damit das Ventil zu öffnen. Dabei ist dies nur dann möglich, wenn die durch den Schaltstift aufgebrachte Kraft größer ist als die zu überwindende Haltekraft, die durch die erhöhte Reibung entsteht, da vom Fluid eine Gesamtkraft auf den Ventilkörper ausgeübt wird. Durch geschickte Wahl von Materialien, Geometrien der beteiligten Flächen und der Ausgestaltung des Ventils lässt sich in diesem Fall erreichen, dass das Ventil nur dann geöffnet werden kann, wenn kein oder nur ein geringer Druck anliegt, was in der Regel dann der Fall ist, wenn sich das Prothesenkniegelenk gestreckt ist und keine beugende Bewegung stattfindet.

Vorzugsweise verfügt das Ventil über eine Schaltfeder, die eingerichtet, ist, eine Kraft von dem Schaltstift auf den Ventilkörper zu übertragen, wenn der Schaltstift betätigt wird. Dadurch kann die aufgebrachte Kraft nach oben begrenzt werden. Dabei ist wichtig, dass sich die Schaltfeder im Kraftfluss der Ansteuerung befindet. Sie kann beispielsweise als Unterbrechung im Schaltstift oder im Schalthebel oder an anderer Stelle im Kraftfluss zwischen dem Unterteil und dem Ventilkörper angeordnet sein. Unabhängig davon, wie stark die auf dem Schaltstift aufgebrachte Kraft ist, wird über die Feder zumindest kurzfristig nur eine sehr begrenzte Kraft übertragen, die beispielsweise durch geschickte Wahl der Federkonstante, der Art der Feder und/oder des Materials der Feder so klein ist, dass zum Öffnen des Ventils, also zum Verschieben des Ventilkörpers von der ersten Position in die zweite Position nicht ausreicht, sofern der Druck des einströmenden Fluides größer als ein vorbestimmter Grenzwert, beispielsweise größer als 1 bar ist. Diese Ausgestaltung ist insbesondere dann von Vorteil, wenn auf den Schaltstiften selbst größere Kräfte einwirken können.

Vorteilhafterweise ist die Feder derart ausgewählt, dass die von der Schaltfeder auf den Ventilkörper übertragene Kraft nicht ausreicht, den Ventilkörper von der ersten Position in die zweite Position zu bringen, wenn der Ventilkörper von einem Fluid mit einem Druck von 1 bar durch den Zulauf angeströmt wird.

In einer bevorzugten Ausführungsform ist der Ventilkörper in Richtung auf die erste Position federbelastet. Auch dadurch kann selbstverständlich die Kraft zumindest mit eingestellt werden, die benötigt wird, um den Ventilkörper von der ersten Position in die zweite Position zu bringen und dadurch das Ventil zu öffnen. Dabei ist zu beachten, dass diese Kraft, die durch die Federbelastung des Ventilkörpers in Richtung auf die erste Position hervorgerufen wird, unabhängig von der Größe des Druckes ist, der durch das Einströmen des Fluids hervorgerufen wird. Die Federbelastung wird vorzugsweise durch eine Feder, beispielsweise eine Schraubenfeder hervorgerufen und dient insbesondere dazu, das Ventil zu schließen, wenn sich die Bewegung des Gelenks umdreht oder das Ventil nicht von Fluid durchströmt wird.

Vorzugsweise verfügt das Ventil über einen Drosselablauf, wobei der Ventilkörper derart ausgebildet und angeordnet ist, dass durch den Zulauf einströmendes Fluid das Ventil zumindest auch durch den Drosselablauf verlässt, unabhängig von der Position des Ventilkörpers. Auf diese Weise wird verhindert, dass das Ventil eine Hydraulikverbindung oder Hydraulikleitung, in die es eingesetzt ist, vollständig blockieren kann. Es wird lediglich im geschlossenen Zustand des Ventils, wenn sich also der Ventilkörper in der ersten Position befindet, ein erhöhter Strömungswiderstand dem Fluid entgegengesetzt, als im geöffneten Zustand des Ventils, wenn folglich der Ventilkörper sich in der zweiten Position befindet. Die Stärke des Strömungswiderstandes ist vorzugsweise einstellbar. So kann sie auf den Träger der Prothese eingestellt werden, um beispielsweise ein bequemes und komfortables Treppabgehen zu ermöglichen.

Die Erfindung löst die gestellte Aufgabe folglich durch ein Prothesenkniegelenk mit einer Hydraulikanordnung, die eine Extensionskammer, eine Flexionskammer und ein Ventil der hier beschriebenen Art aufweist, wobei der Zulauf des Ventils mit der Flexionskammer und der Ablauf des Ventils mit der Extensionskammer verbunden sind. Wird ein derartiges Prothesenkniegelenk gebeugt, strömt das Hydraulikfluid von der Flexionskammer in die Extensionskammer. Daher ist die Flexionskammer mit dem Zulauf des Ventils verbunden. Dadurch wird erreicht, dass das Ventil nicht geöffnet werden kann, also der Ventilkörper nicht von der ersten Position in die zweite Position verschoben werden kann, solange das belastete Knie nicht vollständig oder zumindest nahezu vollständig gestreckt ist. In diesem Fall wird nämlich durch das erzeugte Drehmoment ein Druck auf das Fluid in der Flexionskammer ausgeübt, der sich in einer Gesamtkraft auf den Ventilkörper innerhalb des Ventils niederschlägt.

In einer bevorzugten Ausgestaltung des Prothesenkniegelenkes verfügt das Prothesenkniegelenk über ein Unterteil, das mit einem Unterschenkel verbindbar ist. Das Unterteil verfügt über ein erstes Bauteil und ein zweites Bauteil, die um eine Steuerachse schwenkbar aneinander gelagert sind. Zudem verfügt das Unterteil über eine Schaltmechanik, die derart ausgebildet und angeordnet ist, dass der Schaltstift des Ventils betätigt wird, wenn auf das Unterteil eine Kraft wirkt, deren Wirklinie vor der Steuerachse verläuft. Eine solche Kraft entsteht immer dann, wenn eine Vorfußbelastung eines Prothesenfußes, der unterhalb des Prothesenkniegelenks angeordnet ist, auftritt. Durch diese Ausgestaltung wird ein vollständig ohne elektronische Bauteile steuerbares Prothesenkniegelenk erreicht, das von einem geschlossenen Zustand, in dem es einen erhöhten Strömungswiderstand dem Hydraulikfluid entgegenstellt, in einen geöffneten Zustand gebracht wird, sobald eine Vorfußbelastung vorliegt, und der Druck des Fluids in der Flexionskammer so klein ist, dass die durch den Druck gerufene Haltekraft zwischen dem Ventilkörper und der Gehäusewand des Ventils überwunden werden kann. Dadurch werden ohne elektronische Bauteile die aus dem Stand der Technik nur elektronisch realisierten Kniegelenke ermöglicht.

Mit Hilfe der beiliegenden Zeichnungen wird nachfolgend ein Ausführungsbeispiel der vorliegenden Erfindung näher erläutert. Es zeigen
- Figuren 1, 3, 5 und 7 -: schematische Schnittdarstellungen durch ein Prothesenkniegelenk in unterschiedlichen Situationen,
- Figuren 2, 4, 6 und 8 -: vergrößerte Ausschnitte aus den jeweils vorangegangenen Figuren,
- Figur 9 -: die schematische Darstellung einer weiteren Ausführungsform der vorliegenden Erfindung und
- Figuren 10 und 11 -: schematische Ansichten eines Ventils gemäß einem Ausführungsbeispiel der vorliegenden Erfindung.

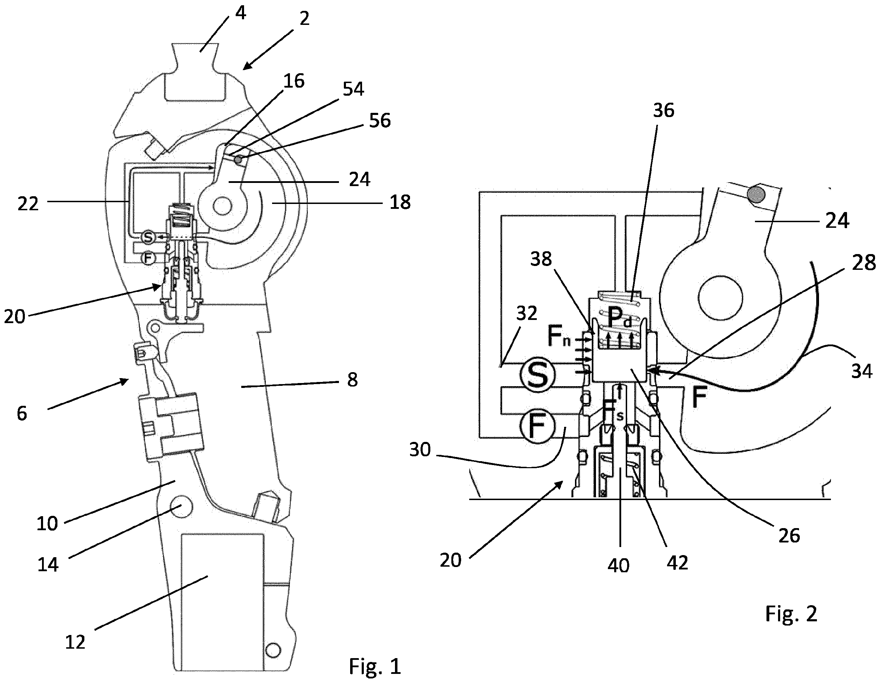

Figur 1 zeigt eine Schnittdarstellung durch ein Prothesenkniegelenk gemäß einem ersten Ausführungsbeispiel der vorliegenden Erfindung. Es verfügt über ein Oberteil 2 mit einem oberen Befestigungselement 4, an dem beispielsweise ein Prothesenschaft oder ein anderes Prothesenbauteil angeordnet werden kann. Das Oberteil 2 ist verschwenkbar an einem Unterteil 6 angeordnet, das ein erstes Bauteil 8 und ein zweites Bauteil 10 aufweist, an dem sich ein unteres Befestigungselement 12 befindet, das im gezeigten Ausführungsbeispiel als Rohrklemmung für ein Unterschenkelrohr ausgebildet ist. Das erste Bauteil 8 ist am zweiten Bauteil 10 um eine Steuerachse 14 schwenkbar angeordnet.

In dem Prothesenkniegelenk befindet sich eine Hydraulikanordnung mit einer Extensionskammer 16, einer Flexionskammer 18 und einem Ventil 20, das in einer Fluidverbindung 22 angeordnet ist, durch die die Extensionskammer 16 mit der Flexionskammer 18 verbunden ist. Die Extensionskammer 16 ist von der Flexionskammer 18 durch einen Kolben 24 getrennt, der sich in dem Volumen, das durch die Extensionskammer 16 und die Flexionskammer 18 gebildet wird, bewegt, wenn das Knie bewegt wird, also das Oberteil 2 relativ zu dem Unterteil 6 verschwenkt wird. Dadurch wird Fluid, das sich in dem Hohlraum befindet, von der Extensionskammer 16 in die Flexionskammer 18 oder umgekehrt geleitet. Im gezeigten Ausführungsbeispiel ist in dem Kolben 24 eine Bohrung 54 dargestellt, in der sich ein Rückschlagventil 56 befindet. Diese Bohrung 54 stellt eine weitere Fluidverbindung zwischen der Extensionskammer 16 und der Flexionskammer 18 dar, durch den Fluidfluss aus der Extensionskammer 16 in die Flexionskammer 18 erlaubt, in die entgegengesetzte Richtung jedoch verhindert wird. Die Stellung des schematisch dargestellten Rückschlagventils 56 entspricht dabei in den gezeigten Figuren nicht der tatsächlichen Stellung, sondern soll nur die Anordnung des Rückschlagventils 56 illustrieren.

Figur 2 zeigt einen vergrößerten Ausschnitt. Das Ventil 20 verfügt über einen Ventilkörper 26, der sich in der in Figur 2 gezeigten Situation in der ersten Position befindet. Das Ventil verfügt über einen Zulauf 28, einen Ablauf 30 sowie einen Drosselablauf 32. Ein Hydraulikfluid, das entlang des Pfeils 34 durch den Zulauf 28 in das Ventil eintritt, kann nicht durch den Ablauf 30 das Ventil verlassen, da sich der Ventilkörper 26 in der ersten Position befindet. In diese Position ist er durch eine Feder 36 federbelastet. Das Fluid kann jedoch im gezeigten Ausführungsbeispiel durch den Ventilkörper 26 hindurchtreten und durch den Drosselablauf 32 das Ventil verlassen. Einer Verschwenkung des Kolbens 24 im Uhrzeigersinn wird folglich ein großer Strömungswiderstand entgegengestellt.

Das Fluid, das durch den Zulauf 28 in das Ventil eindringt, trifft auf den Ventilkörper 26 und übt eine Gesamtkraft auf diesen Ventilkörper 26 aus, die zumindest auch eine Komponente aufweist, die senkrecht auf einer Verschieberichtung steht, entlang derer der Ventilkörper 26 verschoben werden kann. Die Verschieberichtung verläuft in den gezeigten Darstellungen von oben nach unten. Durch die aufgebrachte Gesamtkraft entsteht an der gegenüberliegenden Seite, an der der Ventilkörper 26 an einer Gehäusewand 38 anliegt, eine Gegenkraft Fₙ. Das Ventil 20 verfügt zudem über einen Schaltstift 40, der im gezeigten Ausführungsbeispiel von unten eine Schaltkraft Fₛ auf den Ventilkörper 26 ausüben kann, um diesen aus der gezeigten ersten Position in die zweite Position zu verschieben. Dabei entsteht aufgrund der Haftreibung aus der Gegenkraft Fₙ eine Reibungskraft Fᵣ, die sich aus dem Produkt der Gegenkraft Fₙ mit dem Haftreibungskoeffizienten µ ergibt.

Um den Schaltstift herum ist eine Rückführfeder 42 angeordnet, durch die der Schaltstift 40 wieder in die Ausgangsposition gebracht wird, nachdem ein Schaltvorgang abgeschlossen ist.

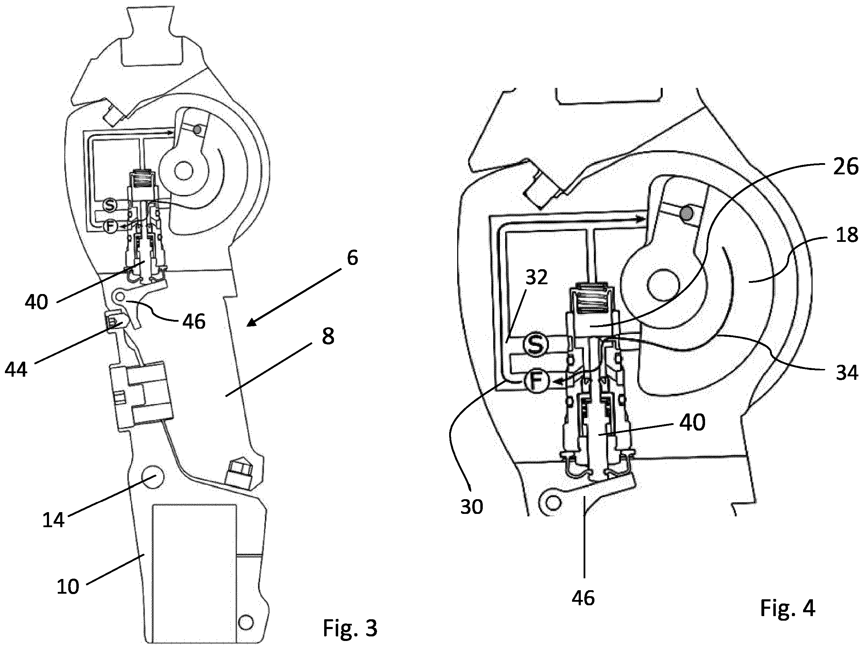

Figur 3 zeigt das Prothesenkniegelenk aus Figur 1 in einer zweiten Situation. Durch beispielsweise eine Vorfußlast, also eine auf das Unterteil 6 wirkende Kraft, deren Wirklinie vor der Steuerachse 14 liegt, im gezeigten Ausführungsbeispiel also links von der Steuerachse 14, wird das erste Bauteil 8 relativ zum zweiten Bauteil 10 um die Steuerachse 14 herum verschwenkt. Dabei wird über eine Schaltschraube 44 ein Schalthebel 46 bewegt, der von unten an dem Schaltstift 40 anstößt und so eine Schaltkraft auf den Schaltstift 40 überträgt.

Figur 4 zeigt einen vergrößerten Ausschnitt. Man erkennt den Schalthebel 46, der den Schaltstift 40 nach oben verschoben hat, wodurch der Ventilkörper 26 aus der ersten Position, die in den Figuren 1 und 2 gezeigt war, in die zweite Position verschoben wurde. Das Fluid kann nun aus der Flexionskammer 18 entlang des Pfeils 34 durch das Ventil hindurchströmen und nicht nur durch den Drosselablauf 32, sondern auch durch den Ablauf 30 das Ventil verlassen. Der Dämpfungswiderstand, der dem Fluid entgegengestellt wird, ist folglich deutlich geringer als in der Situation in Figur 1 und 2.

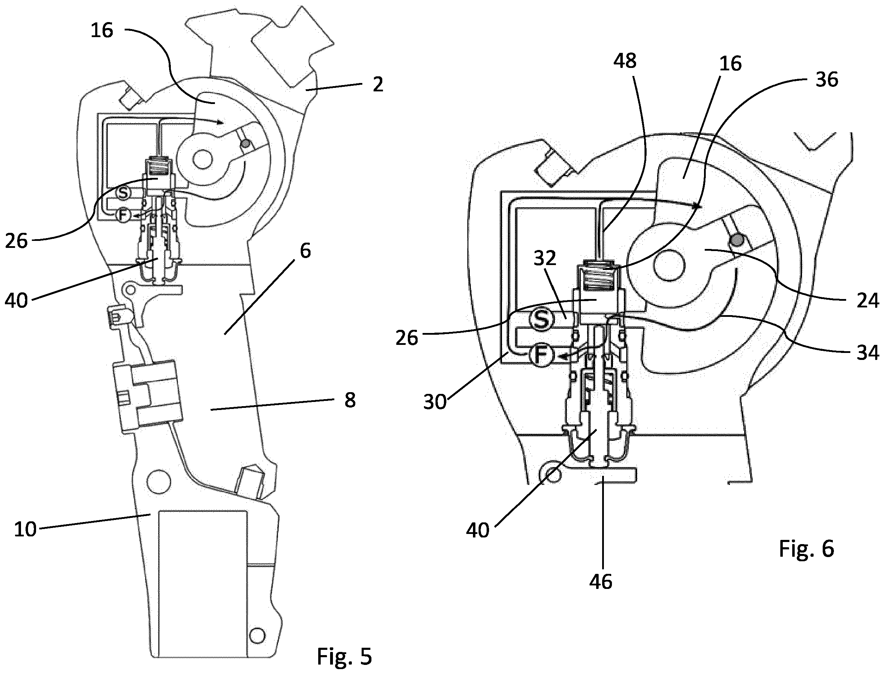

Figur 5 zeigt das Prothesenkniegelenk in einer weiteren Situation. Man erkennt, dass das Oberteil 2 relativ zum Unterteil 6 verschwenkt wurde. Die Extensionskammer 16 ist deutlich vergrößert. Allerdings ist das erste Bauteil 8 relativ zum zweiten Bauteil 10 des Unterteils 6 nicht mehr verschwenkt und der Schaltstift 40 ist in seine ursprüngliche Position zurückgekehrt. Dennoch verbleibt der Ventilkörper 26 in der zweiten Position.

Figur 6 zeigt einen vergrößerten Ausschnitt. Der Schalthebel 46 ist in die Position aus Figur 1 zurückgekehrt und auch der Schaltstift 40 ist nicht mehr in Kontakt mit einer Unterseite des Ventilkörpers 26. Durch das Verschwenken des Oberteils 2 relativ zum Unterteil 6 des Prothesenkniegelenkes kommt es jedoch zu einer Verschwenkung des Kolbens 24, wodurch Fluid entlang des Pfeils 34 durch das Ventil hindurchgedrückt wird. Innerhalb des Ventils 20 entsteht ein Überdruck, da durch den Ablauf 30 und den Drosselablauf 32 ein Strömungswiderstand entgegengesetzt wird.

Oberhalb der Feder 36 befindet sich ein Verbindungskanal 48, der das Volumen, in dem sich die Feder 36 befindet, mit der Extensionskammer 16 verbindet. In diesem Bereich befindet sich ein Unterdruck des Hydraulikfluids. Da sich unterhalb des Ventilkörpers 26 ein Überdruck und oberhalb des Ventilkörpers 26 ein Unterdruck befindet, wird der Ventilkörper 26 auch gegen die Federkraft der Feder 36 in der in den Figuren 5 und 6 gezeigten zweiten Position gehalten, auch wenn die Schaltkraft Fₛ, die durch den Schaltstift 40 aufgebracht wurde, bereits nicht mehr vorhanden ist. Die Flexion des Knies ist folglich mit nur relativ geringem Strömungswiderstand und damit geringer Dämpfung möglich.

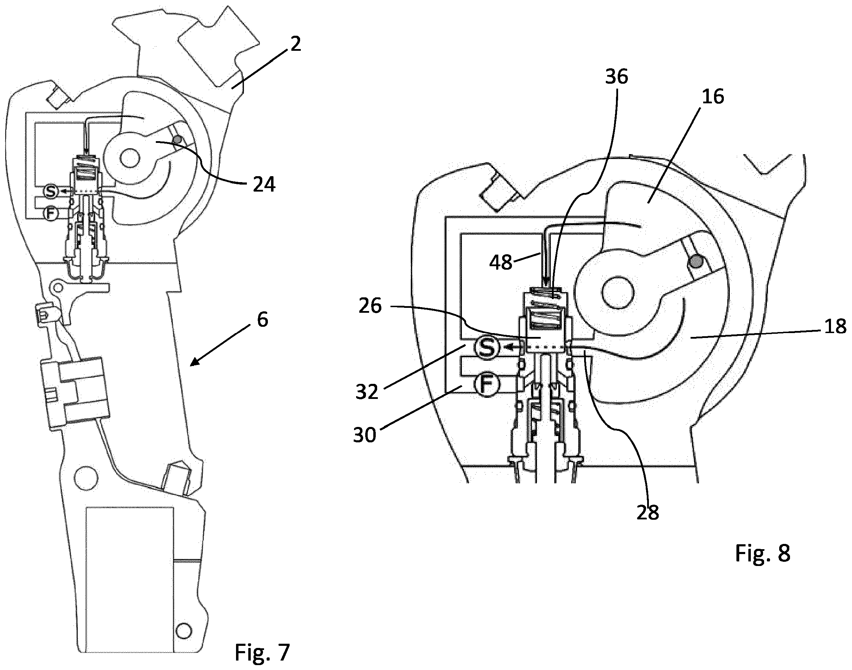

Figur 7 zeigt die Situation bei der Bewegungsumkehr. Dabei soll das Oberteil 2 relativ zum Unterteil 6 so verschwenkt werden, dass das Knie gestreckt wird. Dies hat eine Bewegung des Kolbens 24 entgegen den Uhrzeigersinn zur Folge.

Figur 8 zeigt einen vergrößerten Ausschnitt, aus dem zu erkennen ist, dass wegen des durch die Bewegungsumkehr hervorgerufenen Überdruckes in der Extensionskammer 16 dieser Druck auch durch den Verbindungskanal 48 in das Volumen weitergegeben wird, in dem sich die Feder 36 befindet. Dadurch wird die in Figur 6 vorhandene Drucksituation, die dafür sorgt, dass der Ventilkörper 26 in der gezeigten zweiten Position verbleibt, nicht mehr vorhanden ist. Vielmehr drückt die Feder 36 und der Überdruck, der durch den Verbindungskanal 48 aufgebracht wird, den Ventilkörper 26 zurück in die erste Position, in der Fluid nicht mehr durch den Ablauf 30, sondern nur noch durch den Drosselablauf 32 in das Ventil selbst eintreten kann, das es durch den Zulauf 28 in die Flexionskammer 18 verlässt.

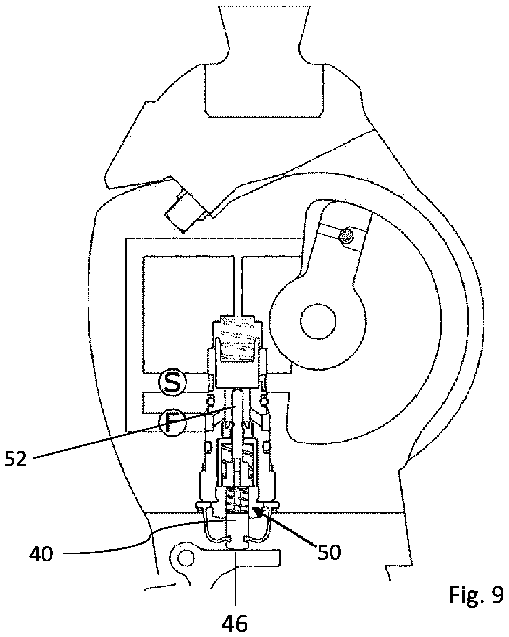

Figur 9 zeigt eine vergrößerte Darstellung der Ventilanordnung. Insbesondere ist eine Schaltfeder 50 dargestellt, die zwischen dem Schaltstift 40 und einem Übertragungsstift 52 angeordnet ist. Der Schalthebel 46 trifft auf den Schaltstift 40, der dadurch bewegt wird. Diese Bewegung wird jedoch nicht unmittelbar auf den Ventilkörper 26 übertragen, sondern zunächst von dem Schaltstift 40 auf die Schaltfeder 50 übertragen. Erst diese überträgt die Schaltkraft auf den Übertragungsstift 52 und dadurch auf den Ventilkörper 26.

Durch die Wahl der Federkonstante der Schaltfeder 50 ist die Kraft zumindest kurzfristig nach oben begrenzt. Die über die Schaltfeder 50 auf den Ventilkörper 26 aufbringbare Kraft zum Schalten des Ventils ist dabei so gewählt, dass sie ausreichend ist, den Federdruck P_{d} zu überwinden und den Ventilkörper 26 aus der gezeigten ersten Position in die zweite Position zu verschieben, wenn keine oder nur eine sehr geringe Gegenkraft Fₙ vorliegt. Wird durch das einströmende Fluid jedoch eine Gesamtkraft auf den Ventilkörper 26 ausgeübt und dadurch die Gegenkraft Fₙ hervorgerufen, ist die Schaltkraft Fₛ, die durch die Schaltfeder 50 und den Schaltstift 40 auf den Ventilkörper 26 übertragen wird, nicht ausreichend, um das Ventil zu schalten.

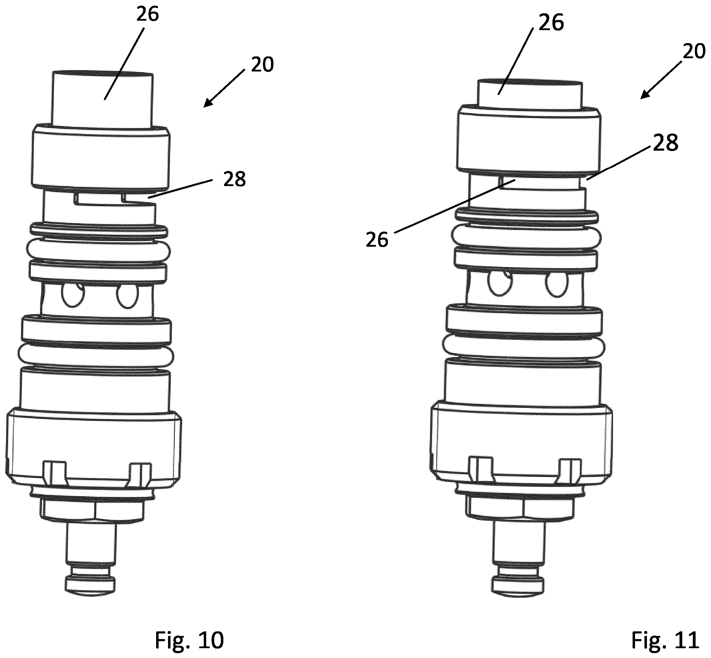

Figuren 10 und 11 zeigen eine isolierte Darstellung des Ventils 20. Während sich der Ventilkörper 26 in der Darstellung in Figur 10 in der zweiten Position befindet und daher der Zulauf 28 frei ist, ist dies in Figur 11 anders. Der Ventilkörper 26 befindet sich in der ersten Position, so dass ein Fluid, das durch den Zulauf 28 in das Ventil 20 eintritt, auf den Ventilkörper 26 trifft und eine Kraft ausübt. Man erkennt, dass der Zulauf 28 über einen Abschnitt des Umfanges des Ventils und damit auch den Ventilkörpers 26 erfolgt, der im gezeigten Ausführungsbeispiel kleiner ist als 180°.

### Bezugszeichenliste

Fₙ Gegenkraft
Fₛ Schaltkraft
Fᵣ Reibungskraft
2 Oberteil
4 oberes Befestigungselement
6 Unterteil
8 erstes Bauteil
10 zweites Bauteil
12 unteres Befestigungselement
14 Steuerachse
16 Extensionskammer
18 Flexionskammer
20 Ventil
22 Fluidverbindung
24 Kolben
26 Ventilkörper
28 Zulauf
30 Ablauf
32 Drosselablauf
34 Pfeil
36 Feder
38 Gehäusewand
40 Schaltstift
42 Rückführfeder
44 Schaltschraube
46 Schalthebel
48 Verbindungskanal
50 Schaltfeder
52 Übertragungsstift
54 Bohrung
56 Rückschlagventil

## Patentansprüche

1. Prothesenkniegelenk mit einer Hydraulikanordnung, die eine Extensionskammer (16), eine Flexionskammer (18) und ein Ventil (20) aufweist, das
- einen Zulauf (28),
- einen Ablauf (30), der mit dem Zulauf (28) durch eine Fluidverbindung verbunden ist, und
- einen Ventilkörper (26) aufweist, der durch Verschieben entlang einer Verschieberichtung
∘ in eine erste Position, in der die Fluidverbindung blockiert ist, und
∘ in eine zweite Position bringbar ist, in der die Fluidverbindung geöffnet ist,
wobei der Zulauf (28) des Ventils (20) mit der Flexionskammer (18) und der Ablauf (30) des Ventils (20) mit der Extensionskammer (16) verbunden sind, **dadurch gekennzeichnet, dass** der Zulauf (28) derart ausgebildet und angeordnet ist, dass ein durch den Zulauf (28) eintretendes Fluid eine Gesamtkraft auf den Ventilkörper (26) ausübt, die zumindest auch in einer Kraftrichtung wirkt, die senkrecht auf der Verschieberichtung steht, wenn sich der Ventilkörper (26) in der ersten Position befindet.

2. Prothesenkniegelenk nach Anspruch 1, **dadurch gekennzeichnet, dass** der Ventilkörper (26) in Kraftrichtung an einer Gehäusewand (38) anliegt.

3. Prothesenkniegelenk nach Anspruch 1 oder 2, **dadurch gekennzeichnet, dass** der Zulauf (28) derart ausgebildet ist, dass eintretendes Fluid den Ventilkörper (26) entlang eines Abschnittes seines Umfanges anströmt, der kleiner als 240°, bevorzugt kleiner als 210° und größer als 120°, bevorzugt größer als 150° und besonders bevorzugt gleich 180° ist.

4. Prothesenkniegelenk nach einem der vorstehenden Ansprüche, **dadurch gekennzeichnet, dass** das Ventil (20) einen Schaltstift (40) aufweist, durch dessen Betätigen der Ventilkörper (26) von der ersten Position in die zweite Position bringbar ist.

5. Prothesenkniegelenk nach Anspruch 4 **dadurch gekennzeichnet, dass** das Ventil (20) eine Schaltfeder (50) aufweist, die eingerichtet ist, eine Kraft von dem Schaltstift (40) auf den Ventilkörper (26) zu übertragen, wenn der Schaltstift (40) betätigt wird.

6. Prothesenkniegelenk nach Anspruch 5, **dadurch gekennzeichnet, dass** die Schaltfeder (50) derart ausgewählt ist, dass die von der Schaltfeder (50) auf den Ventilkörper (26) übertragene Kraft nicht ausreicht, den Ventilkörper (26) von der ersten Position in die zweite Position zu bringen, wenn der Ventilkörper (26) von einem Fluid mit einem Druck von 1 bar durch den Zulauf angeströmt wird.

7. Prothesenkniegelenk nach einem der vorstehenden Ansprüche, **dadurch gekennzeichnet, dass** der Ventilkörper (26) in Richtung auf die erste Position federbelastet ist.

8. Prothesenkniegelenk nach einem der vorstehenden Ansprüche, **dadurch gekennzeichnet, dass** das Ventil (20) einen Drosselablauf (32) aufweist, wobei der Ventilkörper (26) derart ausgebildet und angeordnet ist, dass durch den Zulauf (28) einströmendes Fluid das Ventil (20) zumindest auch durch den Drosselablauf (32) verlässt, unabhängig von der Position des Ventilkörpers (26).

9. Prothesenkniegelenk nach einem der vorstehenden Ansprüche, **dadurch gekennzeichnet, dass** das Prothesenkniegelenk ein Oberteil und ein Unterteil aufweist und die Schaltfeder im Kraftfluss zwischen dem Unterteil und dem Ventil angeordnet ist.

10. Prothesenkniegelenk nach einem der vorstehenden Ansprüche, **dadurch gekennzeichnet, dass** das Prothesenkniegelenk ein Unterteil (6) aufweist, das ein erstes Bauteil (8) und ein zweites Bauteil (10) aufweist, die um eine Steuerachse (12) schwenkbar aneinander gelagert sind und eine Schaltmechanik aufweisen, die derart ausgebildet und angeordnet ist, dass der Schaltstift (40) des Ventils betätigt wird, wenn auf das Unterteil (6) eine Kraft wirkt, deren Wirklinie vor der Steuerachse (12) verläuft.

## Claims

1. A prosthetic knee joint with a hydraulic arrangement that comprises an extension chamber (16), a flexion chamber (18) and a valve (20) with
- an inlet (28)
- an outlet (30) that is connected to the inlet (28) via a fluid connection, and
- a valve body (26), which can be brought - by displacing it along a displacement direction -
∘ into a first position, in which the fluid connection is blocked, and
∘ a second position, in which the fluid connection is open,
wherein the inlet (28) of the valve (20) is connected to the flexion chamber (18) and the outlet (30) of the valve (20) is connected to the extension chamber (16),
**characterized in that** the inlet (28) is designed and arranged in such a way that a fluid entering through the inlet (28) exerts a total force on the valve body (26) that at least also acts in a force direction which is perpendicular to the displacement direction when the valve body (26) is in the first position.

2. The prosthetic knee joint according to claim 1, **characterized in that** the valve body (26) rests on a housing wall (38) in the direction of force.

3. The prosthetic knee joint according to claim 1 or 2, **characterized in that** the inlet (28) is designed such that incoming fluid flows towards the valve body (26) along a section of its circumference which is smaller than 240°, preferably smaller than 210°, and greater than 120°, preferably greater than 150°, and is especially preferably 180°.

4. The prosthetic knee joint according to one of the above claims, **characterized in that** the valve (20) comprises a switch pin (40), the activation of which enables the movement of the valve body (26) from the first position into the second position.

5. The prosthetic knee joint according to claim 4, **characterized in that** the valve (20) comprises a switch spring (50), which is configured to transfer a force from the switch pin (40) to the valve body (26) when the switch pin (40) is activated.

6. The prosthetic knee joint according to claim 5, **characterized in that** the switch spring (50) is selected such that the force transferred from the switch spring (50) to the valve body (26) is not sufficient to move the valve body (26) from the first position into the second position when the valve body (26) is subjected to a flow of fluid via the inlet, the fluid being at a pressure of 1 bar.

7. The prosthetic knee joint according to one of the preceding claims, **characterized in that** the valve body (26) is spring-loaded towards the first position.

8. The prosthetic knee joint according to one of the preceding claims, **characterized in that** the valve (20) comprises a throttle outlet (32), wherein the valve body (26) is designed and arranged in such a way that fluid flowing in through the inlet (28) leaves the valve (20) at least also through the throttle outlet (32), regardless of the position of the valve body (26).

9. The prosthetic knee joint according to one of the preceding claims, **characterized in that** the prosthetic knee joint features an upper part and a lower part, and the switch spring is arranged in the flux between the lower part and the valve.

10. The prosthetic knee joint according to one of the preceding claims, **characterized in that** the prosthetic knee joint comprises a lower part (6) with a first component (8) and a second component (10), which are mounted to one another such that they can be swivelled about a control axis (12) and which feature a switch mechanism, which is designed and arranged such that the switch pin (40) of the valve is activated when a force acts on the lower part (6), the pitch line of said force extending in front of the control axis (12).

## Revendications

1. Prothèse de l'articulation du genou, comportant un ensemble hydraulique qui comprend une chambre d'extension (16), une chambre de flexion (18), et une valve (20) munie
- d'une entrée (28),
- d'une sortie (30) reliée à l'entrée (28) par une communication fluidique, et
- d'un corps de valve (26) qui, par translation le long d'une direction de translation, peut être amené
• dans une première position dans laquelle la communication fluidique est bloquée, et
• dans une deuxième position dans laquelle la communication fluidique est ouverte,
l'entrée (28) de la valve (20) étant reliée à la chambre de flexion (18), et la sortie (30) de la valve (20) étant reliée à la chambre d'extension (16),
**caractérisée en ce que**
l'entrée (28) est réalisée et mise en place de telle sorte qu'un fluide, entrant par l'entrée (28), exerce une force totale sur le corps de valve (26), qui agit au moins également dans une direction de force perpendiculaire à la direction de translation, lorsque le corps de valve (26) est dans la première position.

2. Prothèse de l'articulation du genou selon la revendication 1, **caractérisée en ce que** le corps de valve (26) s'appuie contre une paroi de boîtier (38) dans la direction de la force.

3. Prothèse de l'articulation du genou selon la revendication 1 ou 2, **caractérisée en ce que** l'entrée (28) est réalisée de telle sorte que le fluide entrant attaque le corps de valve (26) le long d'une portion de sa circonférence qui est inférieure à 240°, de préférence inférieure à 210°, et supérieure à 120°, de préférence supérieure à 150° et de manière particulièrement préférée égale à 180°.

4. Prothèse de l'articulation du genou selon l'une des revendications précédentes,
**caractérisée en ce que** la valve (20) comporte une tige de commutation (40) dont l'actionnement permet de faire passer le corps de valve (26) de la première position à la deuxième position.

5. Prothèse de l'articulation du genou selon la revendication 4,
**caractérisée en ce que** la valve (20) comporte un ressort de commutation (50) qui est conçu pour transmettre une force de la tige de commutation (40) au corps de valve (26), lorsque la tige de commutation (40) est actionnée.

6. Prothèse de l'articulation du genou selon la revendication 5,
**caractérisée en ce que** le ressort de commutation (50) est choisi de telle sorte que la force transmise par le ressort de commutation (50) au corps de valve (26) n'est pas suffisante pour faire passer le corps de valve (26) de la première position à la deuxième position, lorsque le corps de valve (26) est attaqué par un fluide d'une pression de 1 bar à travers l'entrée.

7. Prothèse de l'articulation du genou selon l'une des revendications précédentes,
**caractérisée en ce que** le corps de valve (26) est sollicité par un ressort en direction de la première position.

8. Prothèse de l'articulation du genou selon l'une des revendications précédentes,
**caractérisée en ce que** la valve (20) comporte une sortie d'étranglement (32), le corps de valve (26) étant réalisé et agencé de telle sorte que le fluide entrant par l'entrée (28) quitte la valve (20) au moins également par la sortie d'étranglement (32), quelle que soit la position du corps de valve (26).

9. Prothèse de l'articulation du genou selon l'une des revendications précédentes,
**caractérisée en ce que** la prothèse de l'articulation du genou comporte une partie supérieure et une partie inférieure, et le ressort de commutation est disposé dans le flux de force entre la partie inférieure et la valve.

10. Prothèse de l'articulation du genou selon l'une des revendications précédentes,
**caractérisée en ce que** la prothèse de l'articulation du genou comporte une partie inférieure (6) qui présente un premier composant (8) et un deuxième composant (10) qui sont montés l'un sur l'autre de manière à pouvoir pivoter autour d'un axe de commande (12) et qui présentent un mécanisme de commutation réalisé et disposé de telle sorte que la tige de commutation (40) de la valve est actionnée lorsqu'une force agit sur la partie inférieure (6), dont la ligne d'action s'étend en avant de l'axe de commande (12).
